# EUROPÄISCHE PATENTSCHRIFT

(11) **EP 0 387 595 B1**
(45) Veröffentlichungstag und Bekanntmachung des Hinweises auf die Patenterteilung: **25.05.1994**
(21) Anmeldenummer: 90103779.6
(22) Anmeldetag: 27.02.1990
(51) Int. Cl.: C09B 57/00, C07D 487/04, C07D 471/14, C07D 487/14

(54) **Heterocyclische Verbindungen**
Heterocyclic compounds
Composés hétérocycliques

(30) Priorität: 11.03.1989 DE 3908036; 23.03.1989 DE 3909595
(43) Veröffentlichungstag der Anmeldung: 19.09.1990
(73) Patentinhaber: BAYER AG, 51368 Leverkusen (DE)
(72) Erfinder: Höchstetter, Hans, Dr., D-4000 Düsseldorf (DE)

(56) Entgegenhaltungen:
- EP-A- 0 151 393
- CH-A- 641 457
- US-A- 4 228 168

## Beschreibung

Die Erfindung betrifft heterocyclische Verbindungen der Formel (I) 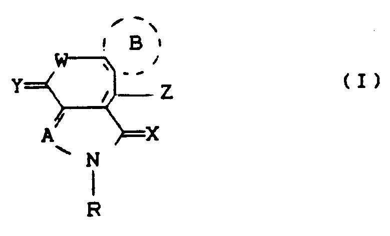
Verfahren zu ihrer Herstellung sowie ihre Verwendung als Farbstoffe oder Pigmente, insbesondere zum Färben oder Pigmentieren von hochmolekularem organischen Material oder als Lackpigmente.

In der Formel (I) bezeichnen:
- R: H, gegebenenfalls substituiertes Alkyl, gegebenenfalls substituiertes Cycloalkyl, gegebenenfalls substituiertes Aryl, gegebenenfalls substituiertes Hetaryl,
- A: C-R¹ oder N
- R¹: gegebenenfalls substituiertes Alkyl, gegebenenfalls substituiertes Cycloalkyl, gegebenenfalls substituiertes Aryl, gegebenenfalls substituiertes Hetaryl,
- X,Y: O, NH,
- Z: Wasserstoff, -CN, gegebenenfalls substituiertes Aryl, gegebenenfalls substituiertes Hetaryl,
- W: O, N-R⁵,
- R⁵: Wasserstoff, gegebenenfalls substituiertes Alkyl, gegebenenfalls substituiertes Cycloalkyl, gegebenenfalls substituiertes Aryl, einen gegebenenfalls substituierten heterocyclischen Rest,
- B: einen gegebenenfalls substituierten carbocyclisch-aromatischen oder heterocyclischen Ring, an die ein oder mehrere weitere Ringe ankondensiert sein können.

Alkyl (R, R¹) steht vorzugsweise für C₁-C₆-Alkyl, besonders bevorzugt für C₁-C₄-Alkyl. Beispielhaft seien genannt: Methyl, Ethyl, n- und i-Propyl, n-, i- und t-Butyl.

Cycloalkyl (R, R¹) steht vorzugsweise für mono-, bi- und tricyclisches C₃-C₁₀-Cycloalkyl, besonders bevorzugt Cyclopropyl, Cyclopentyl, Cyclohexyl.

Die Alkyl- und Cycloalkylreste (R, R¹, W) können z. B. durch Cl, Br, F, -CN, -OH, C₁-C₆-Alkoxy, Mono-C₁-C₄-alkylamino, Di-C₁-C₄-alkylamino, Phenyl oder Naphthyl, die durch Cl, Br, F, C₁-C₆-Alkyl und C₁-C₆-Alkoxy substituiert sein können oder Reste eines 5- oder 6-gliedrigen Ringsystems mit 1 oder 2 Heteroatomen aus der Reihe 0, N, S, an das ein Benzolring ankondensiert sein kann, substituiert sein.

Aryl (R, R¹, Z) steht vorzugsweise für solche carbocyclisch-aromatischen Reste, die 1, 2, 3 oder 4, insbesondere 1 oder 2 Ringe enthalten, z. B. Phenyl, Diphenyl und Naphthyl.

Hetaryl (R, R¹, Z) steht vorzugsweise für solche heterocyclisch-aromatischen Reste, die 1, 2, 3 oder 4, insbesondere 1 oder 2 fünf-, sechs- oder siebengliedrige Ringe enthalten, von denen mindestens einer 1, 2 oder 3, bevorzugt 1 oder 2 Heteroatome aus der Reihe O, N, S enthält.

Als heterocyclisch aromatische Reste seien beispielhaft genannt:
Pyridyl, Pyrimidyl, Pyrazinyl, Triazinyl, Furanyl, Pyrrolyl, Thienyl, Chinolyl, Cumarinyl, Benzofuranyl, Benzimidazolyl, Benzoxazolyl, Dibenzofuranyl, Benzothienyl, Dibanzothienyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalimidyl, Chromonyl, Naphtholactamyl, Benzopyridonyl, ortho-Sulfobenzimidyl, Maleinimidyl, Naphtharidinyl, Benzimidazolonyl, Benzoxazolonyl, Benzthiazolonyl, Benzthiazolinyl, Chinazolonyl, Pyrimidonyl, Chinoxalonyl, Phthalazonyl, Dioxapyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

Die Aryl- und Hetarylreste (R, R¹, Z) können beispielsweise durch Halogen, wie Chlor, Brom, Fluor, CN, R², OR³, SR³, NR⁵R⁶, COOR⁷, NR³COR⁷, NR³COOR⁷, NR³CONR⁵R⁶, -NHSO₂R⁷, -SO₂R⁷, SOR⁷, SO₂OR⁷, CONR⁵R⁶, SO₂NR⁵R⁶, -N=N-R¹⁰, OCOR⁷ und OCONHR⁷ substituiert sein.
- R²: bezeichnet gegebenenfalls substituiertes Alkyl, insbesondere C₁-C₆-Alkyl, besonders bevorzugt C₁-C₄-Alkyl. Als Substituenten seien beispielhaft genannt: Cl, Br, F, -CN, -OCOR⁷, -OR³, -COOR⁷, -SR³, -CONR⁵R⁶, -OCONHR⁷.
- R⁵ und R⁶: bezeichnen Wasserstoff, gegebenenfalls substituiertes Alkyl, insbesondere C₁-C₁₈-Alkyl, vorzugsweise C₁-C₄-Alkyl, gegebenenfalls substituiertes Cycloalkyl, insbesondere Cyclopentyl und Cyclohexyl, gegebenenfalls substituiertes Aryl, insbesondere Phenyl, Naphthyl und Diphenylyl, und einen gegebenenfalls substituierten heterocyclischen Rest, insbesondere den Rest eines 5- oder 6-gliedrigen heterocyclischen Ringes mit 1, 2 oder 3 Heteroatomen aus der Reihe O, N, S, an den ein Benzolring ankondensiert sein kann.

Die Alkyl- und Cycloalkylreste R⁵ und R⁶ können z. B. durch Cl, Br, F, -CN, -OH, Mono-C₁-C₄-alkylamino, Di-C₁-C₄-alkylamino, Phenyl oder Naphthyl, die durch Cl, Br, F, C₁-C₆-Alkyl und C₁-C₆-Alkoxy substituiert sein können oder heterocyclische Reste eines 5- oder 6-gliedrigen heterocyclischen Ringsystems mit 1 oder 2 Heteroatomen aus der Reihe O, N, S, an das ein Benzolring ankondensiert sein kann, substituiert sein.

Auch können R⁵ und R⁶ in der Gruppe -CONR⁵R⁶ zusammen unter Einschluß des N-Atoms einen 5- oder 6-gliedrigen heterocyclischen Ring, z. B. einen Morpholin- oder Piperidinring bilden.

Die Aryl- und Aralkylreste R⁵ und R⁶ können beispielsweise durch Cl, Br, F, C₁-C₁₈-Alkyl, vorzugsweise C₁-C₄-Alkyl, C₁-C₁₈-Alkoxy, vorzugsweise C₁-C₄-Alkoxy, substituiert sein.
- R⁷: bezeichnet Wasserstoff, gegebenenfalls substituiertes Alkyl, vorzugsweise C₁-C₄-Alkyl, gegebenenfalls substituiertes Cycloalkyl, insbesondere Cyclopentyl und Cyclohexyl, gegebenenfalls substituiertes Aralkyl, insbesondere Phenyl- und Naphthyl-C₁-C₄-alkyl, vorzugsweise Benzyl, gegebenenfalls substituiertes Aryl, insbesondere Phenyl und Naphthyl und einen gegebenenfalls substituierten heterocyclischen Rest, insbesondere den Rest eines 5- oder 6-gliedrigen Ringes mit 1, 2 oder 3 Heteroatomen aus der Reihe O, N, S, an den ein Benzolring ankondensiert sein kann.

Die für R⁷ genannten Reste können beispielsweise wie die entsprechenden Reste R⁵ und R⁶ substituiert sein.
- R³: bezeichnet Wasserstoff oder nimmt die Bedeutungen von R⁵ an.
- R¹⁰: bezeichnet den Rest einer Kupplungskomponente, vorzugsweise einer Kupplungskomponente aus der Benzol-, Naphthalin-, Pyrazol- oder Pyridonreihe oder einen gegebenenfalls durch Cl, Br, F, C₁-C₄-Alkyl oder C₁-C₄-Alkoxy substituierten Phenylrest.

Der Ring B in Formel (I) kann ein aromatischer oder heteroaromatischer Ring, der für Aryl (R, R¹, Z) oder Hetaryl (R, R¹, Z) genannten Art sein, der zusätzlich zu den beiden Substituenten an den Anellierungsstellen 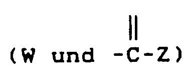
die oben für substituiertes Aryl und Hetaryl aufgeführten Substituenten tragen kann.

Ferner kann B auch für einen nichtaromatischen heterocyclischen Rest stehen, der 1, 2, 3 oder 4, insbesondere 1 oder 2 fünf-, sechs- oder siebengliedrige Ringe enthält, von denen mindestens einer 1, 2, 3 oder 4, bevorzugt 1 oder 2 Heteroatome aus der Reihe O, N, S enthält. Diese heterocyclischen Reste B können wiederum durch gegebenenfalls substituiertes Aryl oder Hetaryl substituiert sein, welche die oben angegebene Bedeutung haben.

Besonders bevorzugte Ringe B sind gegebenenfalls substituierte Aromaten mit oben angeführter Bedeutung und die nachfolgend gezeigten, substituierten Pyrazol-, Pyridin oder Pyrimidinderivate (Anellierungsstellen mit W und C-Z angedeutet) oder ihre tautomeren Formen. 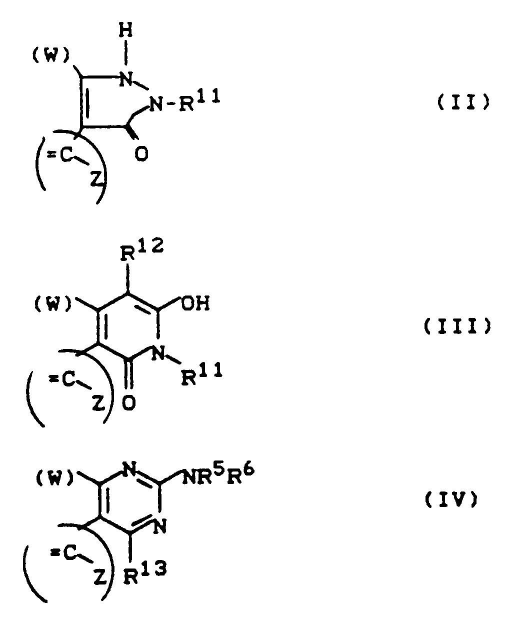
In den Formeln (II), (III) und (IV) bezeichnen:
- R¹¹: aromatischer oder heteroaromatischer Rest von oben angeführter Bedeutung mit oben angeführter Substitution (siehe R, R¹, Z),
- R¹²: -CN, -COOR⁷, -CONR⁵R⁶, wobei R⁵, R⁶ und R⁷ die oben angeführte Bedeutung besitzen,
- R¹³: Halogen oder OR³, wobei R³ die oben angegebene Bedeutung besitzt.

Die Substituenten R⁵ und R⁶ in Formel (IV) besitzen ebenfalls die oben angegebene Bedeutung.

Bevorzugte erfindungsgemäße Verbindungen sind solche der Formel (I), worin W = N-R⁵, wobei R⁵ die oben genannte Bedeutung hat, X = Y = 0, Z = H, -CN, gegebenenfalls substituiertes Aryl, A = N, C-Alkyl, das substituiert sein kann, C = Aryl, das substituiert sein kann und B = substituierter aromatischer Ring, substituierter Pyrazol-, Pyrimidin- oder Pyridinring.

Besonders bevorzugt sind Verbindungen, die in einer ihrer tautomeren Formen den Formeln Va - Vd entsprechen. 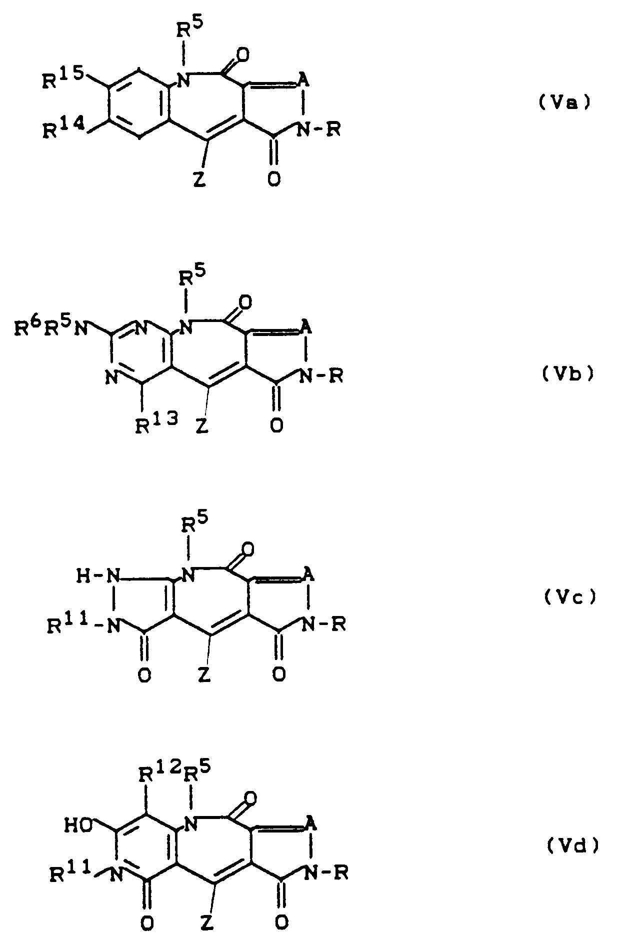
In diesen Formeln haben die Reste R, A, R⁵, R⁶, R¹³, R¹¹, R¹² und Z die oben angegebenen Bedeutungen;
Besonders bevorzugt steht
- R: für Phenyl, das durch 1 oder 2 Substituenten aus der Reihe -SO₃H, Halogen (Cl, Br), -CN, C₁-C₄-Alkyl, -SO₂NH₂ substituiert sein kann.

Neben N steht A besonders bevorzugt für 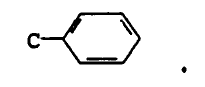
- R¹⁴, R¹⁵: bezeichnen Wasserstoff, Halogen, OR³ oder NR⁵R⁶, wobei R³, R⁵ und R⁶ die angegebenen Bedeutungen besitzen.

Die Verbindungen der Formel I mit Z = H erhält man, indem man entweder Aldehyde der Formel 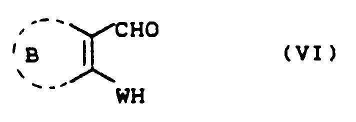
(oder deren funktionelle Derivate, z. B. deren Schiff'sche Basen), worin B und W die oben angegebenen Bedeutungen besitzen, mit Verbindungen der Formel 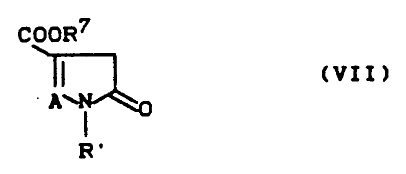
worin R, R⁷ und A die oben angegebenen Bedeutungen besitzen, umsetzt, oder indem man formylierte (und gegebenenfalls als Anil geschützte) Derivate von (VII), nämlich Verbindungen des Typs 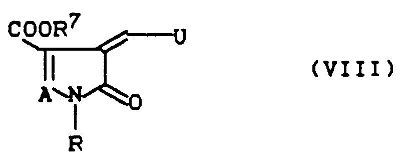
mit C-H-aciden Heterocyclen des Typs 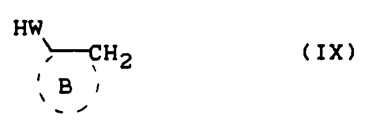
umsetzt. (IX) kann in verschiedenen tautomeren Formen vorliegen.

In den Formeln (VIII) und (IX) bedeuten:
U = OR³ oder NHR⁵; R³, R⁵, R⁷ und R' besitzen die oben angegebenen Bedeutungen.

W und B besitzen ebenfalls die oben angegebenen Bedeutungen, wobei für B bevorzugt heterocyclische Ringe in Frage kommen.

Die CH₂-Gruppe in der Formel (IX) muß durch geeignete Substitution im Ring B so C-H-acide sein, daß sie mit der Aldehydgruppe (oder deren Äquivalent) in (VIII) reagieren kann.

Die genannten Umsetzungen können über Zwischenstufen verlaufen, die unter Protonenwanderung(en) zu einer tautomeren Form der Formel (I) umlagern.

Die Umsetzung erfolgt zweckmäßigerweise unter den bekannten Bedingungen der Knoevenagel-Kondensation, d. h. in einem geeigneten Lösungsmittel in Gegenwart eines sauren oder basischen Katalysators. Geeignete Lösungsmittel sind Alkohole, Glykole, aromatische Kohlenwasserstoffe und aliphatische Carbonsäuren und deren Amide.

Geeignete saure Katalysatoren sind Carbonsäuren, Sulfonsäuren und Aminosäuren, geeignete basische Katalysatoren sind aliphatische Amine. Wird als Aldehyd (VI) oder (VIII) ein entsprechendes Anil (Schiff'sche Base) eingesetzt, so kann bei Durchführung der Reaktion in Alkoholen oder Carbonsäuren auf Zugabe eines Katalysators verzichtet werden.

Die Reaktionstemperaturen liegen zwischen 20 und 150° C, vorzugsweise zwischen 80 und 130° C.

Die Reaktion zu (I) beinhaltet zwei Schritte, nämlich eine Kondensation eines Aldehyds (oder Aldehydäquivalents) mit einer aktiven Methylengruppe sowie eine intramolekulare Veresterung oder Lactambildung (= Reaktion der Gruppe -WH mit der Gruppe CO₂R⁷), die aber in einer Eintopfreaktion ablaufen. Eine Zwischenisolierung eines Primärprodukts ist nicht erforderlich bzw. gar nicht durchführbar.

Die Herstellung von Verbindungen der Formel (I), worin Z = CN, erfolgt beispielsweise dadurch, daß man Verbindungen der Formel (I), worin Z = H, in einem polaren Lösungsmittel wie Dimethylformamid mit Cyanidsalzen umsetzt und gleichzeitig oder anschließend mit einem Oxidationsmittel behandelt. Solche Reaktionen sind prinzipiell bekannt (vergl. DE-A 28 44 299 und DE-A 29 55 546).

Geeignete Cyanidsalze sind NaCN und KCN, geeignete Oxidationsmittel Peroxide, Persulfate, Halogene, Bleitetraacetat und Salpetersäure.

Die Umsetzung mit den Cyaniden erfolgt bei 0 - 120° C, vorzugsweise bei 10 - 40° C.

Die Oxidation wird bei 0 - 20° C durchgeführt.

Verbindungen der Formel (I), worin Z = Aryl oder Hetaryl und W = NH darstellen, werden hergestellt durch Umsetzung von β-Aminoketonen des Typs 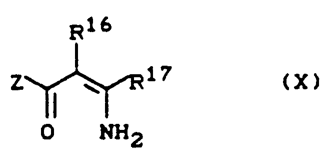
worin
- Z: einen gegebenenfalls substituierten Aromaten oder Heteroaromaten darstellt und
- R¹⁶ und R¹⁷: gemeinsam die Reste eines ankondensierten, gegebenenfalls substituierten Aromaten oder Heteroaromaten darstellen,
mit einem Azolcarbonsäurederivat des Typs (VII).

Verbindungen der Formel (I), worin W = N-Alkyl, werden hergestellt, indem man Verbindungen des Typs I mit W = NH in einem dipolar aprotischen Lösungsmittel wie Dimethylformamid in Gegenwart einer nichtnucleophilen Base wie Kalium- oder Natriumcarbonat mit Alkyltosylaten als N-Alkylierungsmittel umsetzt. Auch solche Reaktionen sind prinzipiell literaturbekannt [z. B. H. Langhals, T. Potrawa, Chem. Ber. 120, 1075 (1987)].

Die als Ausgangsmaterial verwendeten Aldehyde (VI) mit WH=OH oder NH₂ sind bekannt bzw. nach üblichen Methoden leicht zugänglich. Carbocyclische Derivate sind zum Beispiel in DE-A 24 15 661, DE-A 10 98 125 und DE-A 23 63 548 beschrieben.

Andere sind aus literaturbekannten halogenierten Nitrobenzaldehyden leicht zugänglich. Heterocyclische Vertreter von (VI) sind z. B. aus Aminouracilderivaten durch Vilsmeier-Formylierung zugänglich (K. Hirota et. al, Synthesis 1984, 589).

Azolcarbonsäurederivate des Typs (VII) sind ebenfalls in großer Zahl bekannt und gut zugänglich. Pyrrolderivate sind z. B. in EP-A 0 184 981 beschrieben. Pyrazolderivate sind durch Japp-Klingemann-Reaktion von diazotierten Aminen mit Acetbernsteinester in vielfältigem Substitutionsmuster zugänglich (R. Heckendorn, Bull. Soc. Chim. Belg. 95 (1986), 921).

Die Komponenten (VIII) sind aus Azolvorläufern (VII) durch Orthoesterumsetzungen zugänglich [z. B. O. Wolfbeis, Mn. Chem. 112, 369 (1981)].

Als Komponenten (IX) kann man nahezu jeden beliebigen Heterocyclus einsetzen, der die oben näher bezeichnete Gruppe WH und in geeigneter Position eine C-H-acide Methylengruppe besitzt.

Die neuen Substanzen der Formel (I) sind Farbstoffe bzw. Pigmente und eignen sich, je nach Substitution, entweder zum Färben von synthetischen Fasern in gelben bis violetten Tönen oder zum Einsatz als Lack- bzw. Kunststoffpigmente in gelben bis roten Tönen. Manche Farbstoffe besitzen eine sehr gute Thermostabilität und können zum Massefärben von Kunststoffen eingesetzt werden.

Die Verbindungen der Formel (I) mit Z ≠ CN, W = NH und X = Y = O sind gelbe bis rote Substanzen und finden vorzugsweise als Pigmente Verwendung, können je nach ihrer Struktur und der Art der zu färbenden Polymeren jedoch auch als polymerlösliche Farbstoffe für z. B. Polystyrol, Polyamide, ABS, vor allem für lineare Polyester, insbesondere Polyethylenterephthalate eingesetzt werden. Die Verbindungen der Formel (I) fallen in einer für die Pigmentanwendung geeigneten Form an oder können durch an sich bekannte Nachbehandlungsverfahren in die geeignete Form überführt werden, z. B. durch Lösen oder Quellen in starken anorganischen Säuren wie Schwefelsäure und Austragen auf Eis. Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigmentes können durch Variation der Nachbehandlung beeinflußt werden.

Die Verbindungen der Formel (I) eignen sich aufgrund ihrer Licht- und Migrationsechtheit für die verschiedensten Pigmentapplikationen. So können sie zur Herstellung von sehr echt pigmentierten Systemen, in Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z. B. solche mit anorganischen Weißpigmenten wie Titandioxid (Rutil) oder mit Zement verstanden werden. Zübereitungen sind z. B. Flushpasten mit organischen Flüssigkeiten oder Teige und Feinteige mit Wasser, Dispergiermitteln und gegebenenfalls Konservierungsmitteln. Die Bezeichnung Anstrichmittel steht z. B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z. B. Polyethylen oder Polyamide, Superpolyamide, Polymerisate und Mischpolymerisate aus Acrylester, Methacrylestern, Acrylnitril, Acrylamid, Butadien, Styrol sowie Polyurethane und Polycarbonate. Die mit den beanspruchten Produkten pigmentierten Stoffe können in beliebiger Form vorliegen.

Die Verbindungen der Formel (I) mit Z = H, CN und W = O und N-Alkyl eignen sich hervorragend zum Färben von synthetischen Fasern aus z. B. aromatischen Polyestern oder Cellulosetriacetat, die in gelben bis violetten Tönen mit guten Allgemeinechtheiten gefärbt werden.

### Beispiele

In den nachfolgenden Beispielen bedeuten "Teile" Gewichtsteile

### Beispiel 1

9 Teile 1-(2,4-Dichlorphenyl)-3-ethoxycarbonyl-pyrazolon-5 und 11 Teile des durch p-Toluidin geschützten o-Aminobenzaldehyds werden in 300 Teilen Eisessig 5,5 Stunden lang am Rückfluß gekocht. Es wird heiß abgesaugt und mit Eisessig und Methanol bis zum farblosen Ablauf gewaschen. Man erhält 14 Teile der Verbindung 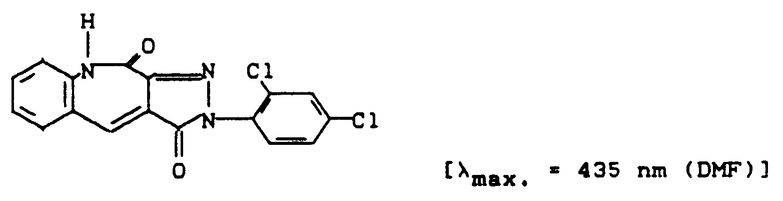
Verfährt man nach den Angaben des Beispiels 1 und setzt entsprechend substituierte Pyrazolonester und Aminobenzaldehydäquivalente ein, so erhält man die in folgender Tabelle aufgeführten Pigmente und Farbstoffe der Formel (XI)

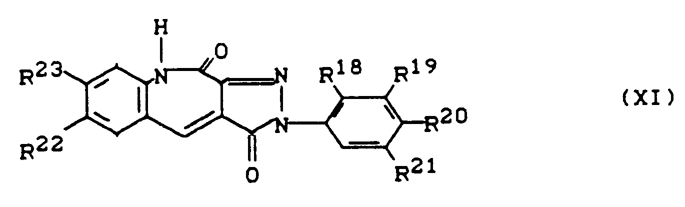

| Bsp. | R¹⁸ | R¹⁹ | R²⁰ | R²¹ | R²² | R²³ | Ausbeute | λₘₐₓ [nm] (DMF) |
|---|---|---|---|---|---|---|---|---|
| 2 | Cl | H | Cl | H | Cl | H | 78 | 440 |
| 3 | Cl | H | H | Cl | Cl | H | 51 | 464 |
| 4 | H | H | Cl | H | Cl | H | 74 | 458 |
| 5 | H | H | Br | H | H | H | 84 | 447 |
| 6 | H | H | Cl | H | H | H | 89 | |
| 7 | H | H | Br | H | Cl | H | 76 | |
| 8 | Cl | H | H | Cl | H | H | 72 | 436 |
| 9 | H | CN | CN | H | H | NMe₂ | 80 | 484, 506 |
| 10 | H | H | t-Butyl | H | H | NEt₂ | 66 | 490 |
| 11 | H | H | Cl | H | H | NMe₂ | 60 | 486 |
| 12 | H | H | SO₂NH₂ | H | H | H | 91 | 442 |

Verfährt man nach den Angaben des Beispiels 1 und setzt statt Pyrazolonderivaten Pyrrolonderivate ein, so erhält man Pyrroloazepinone (orangefarbene Pigmente) für die Beispiel 13 stehen soll.

### Beispiel 13

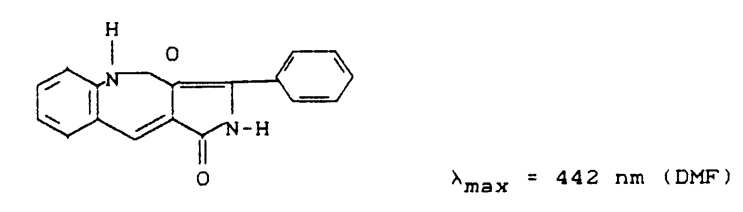

Stellvertretend für solche Vertreter der Formel (I), die als Ring B einen sechsgliedrigen Heterocyclus tragen, sei Beispiel 14 angeführt.

### Beispiel 14

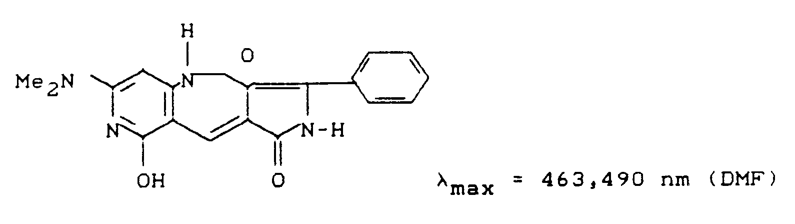

Führt man die in Beispiel 1 beschriebene Reaktion in einem wässrig-alkoholischen Lösungsmittel durch, können auch sulfonsäuregruppenhaltige Pyrazolonderivate eingesetzt werden, was zu wasserlöslichen Farbstoffen führt, wofür Beispiel 15 steht.

### Beispiel 15

färbt Wolle und Polyamid in klarem Orangeton

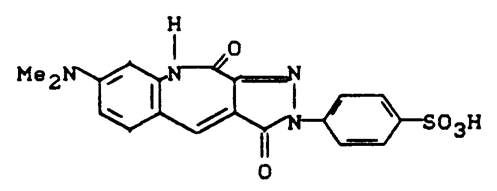

### Beispiel 16

17 Teile der Verbindung 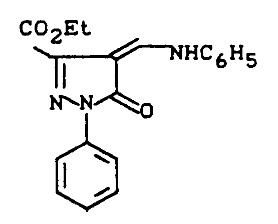
werden mit 9 Teilen der Verbindung 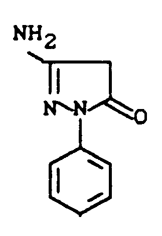
in 100 ml Eisessig 18 h lang am Rückfluß gekocht. Es werden 11 Teile der Verbindung 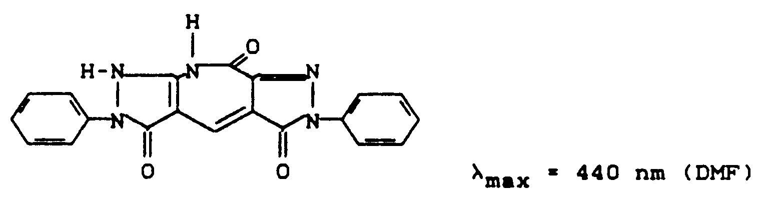
isoliert.

### Beispiel 17 (Anwendungsbeispiel)

4 g feingemahlenes Pigment gemäß Beispiel 6 werden in 92 g eines Einbrennlackes folgender Zusammensetzung dispergiert:
33% Alkydharz
15% Melaminharz
5% Glykolmonomethylether
34% Xylol
13% Butanol
Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden.

Nach erfolgter Dispergierung wird der pigmentierte Lack auf Papier-, Glas-, Kunststoff- oder Metall-Folien auftragen und 30 Minuten bei 130° C eingebrannt. Die Lackierungen besitzen sehr gute Licht- und Wetterbeständigkeit sowie gute Überlackierechtheit.

### Beispiel 18 (Anwendungsbeispiel)

0,2 g Pigment nach Beispiel 6 werden mit 100 g Polyethylen, Polypropylen oder Polystyrolgranulat gemischt. Die Mischung kann entweder bei 220 bis 280° C direkt in einer Spritzgußmaschine verspritzt, oder in einer Strangpresse zu gefärbten Stäben bzw. auf dem Mischwalzwerk zu gefärbten Fellen verarbetet werden. Die Stäbe bzw. Felle werden gegebenenfalls granuliert und in einer Spritzgußmaschine verspritzt.

Die organgefarbenen Formlinge besitzen sehr gute Licht- und Migrationsechtheit. In ähnlicher Weise können bei 280-300° C, gegebenenfalls unter Stickstoffatomosphäre, synthetische Polyamide aus Caprolactam oder Adipinsäure und Hexamethylendiamin oder die Kondensate aus Terephthalsäure und Ethylenglykol gefärbt werden.

## Patentansprüche

1. Heterocyclische Verbindungen der Formel 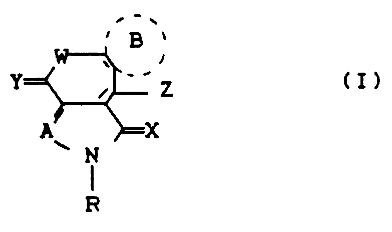 in der
R Wasserstoff, gegebenenfalls substituiertes Alkyl, gegebenenfalls substituiertes Cycloalkyl, gegebenenfalls substituiertes Aryl, gegebenenfalls substituiertes Hetaryl,
A C-R¹, N,
R¹ gegebenenfalls substituiertes Alkyl, gegebenenfalls substituiertes Cycloalkyl, gegebenenfalls substituiertes Aryl, gegebenenfalls substituiertes Hetaryl,
X,Y O, NH,
Z Wasserstoff, -CN, gegebenenfalls substituiertes Aryl, gegebenenfalls substituiertes Hetaryl,
W O, N-R⁵,
R⁵ Wasserstoff, gegebenenfalls substituiertes Alkyl, gegebenenfalls substituiertes Cycloalkyl, gegebenenfalls substituiertes Aryl, einen gegebenenfalls substituierten heterocyclischen Rest und
B einen gegebenenfalls substituierten carbocyclisch-aromatischen oder heterocyclischen Ring, an den ein oder mehrere weitere Ringe ankondensiert sein können, bezeichnen.

2. Verbindungen gemäß Anspruch 1, bei denen X = Y = O, W = N-R⁵, Z = H, -CN, gegebenenfalls substituiertes Aryl, A = N, C-Alkyl, das substituiert sein kann, C-Aryl, das substituiert sein kann und B = substituierter aromatischer Ring, substituierter Pyrazol-, Pyrimidin- oder Pyridinring.

3. Verbindungen gemäß Anspruch 1 oder 2, die in einer ihrer tautomeren Formen der Formel 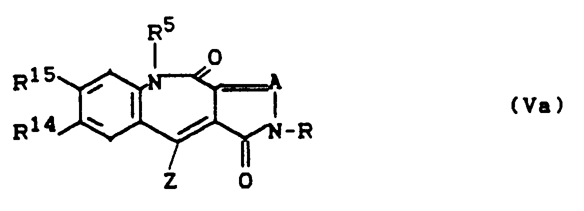 entsprechen,
in der
R¹⁴, R¹⁵ Wasserstoff, Halogen, OR³, NR⁵R⁶ bezeichnen,
R³ für Wasserstoff steht oder die für R⁵ genannten Bedeutungen hat und
R⁶ die für R⁵ genannten Bedeutungen hat.

4. Verbindungen gemäß Anspruch 1 oder 2, die in einer ihrer tautomeren Formen der Formel 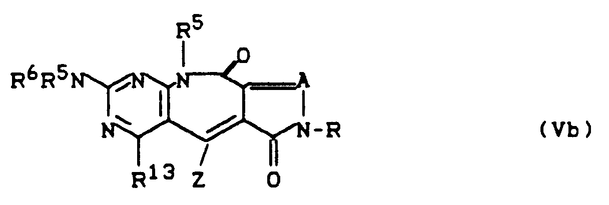 entsprechen,
in der
R⁶ die für R⁵ genannten Bedeutungen hat und
R¹³ Halogen oder OR³ bezeichnet und
R³ für Wasserstoff steht oder die für R⁵ genannten Bedeutungen hat.

5. Verbindungen gemäß Anspruch 1 oder 2, die in einer ihrer tautomeren Formen der Formel 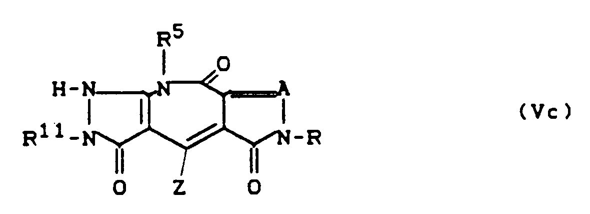 entsprechen
in der
R¹¹ einen gegebenenfalls substituierten Aryl oder einen gegebenenfalls substituieren Hetarylrest bezeichnet.

6. Verbindungen gemäß Anspruch 1 oder 2, die in einer ihrer tautomeren Formen der Formel 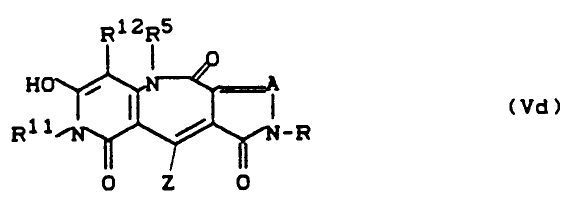 entsprechen,
in der
R¹¹ einen gegebenenfalls substituierten Aryl oder einen gegebenenfalls substituierten Hetarylrest,
R¹² -CN, -COOR⁷, -CONR⁵R⁶ bezeichnen,
R⁶, R⁷ die für R⁵ genannten Bedeutungen haben.

7. Verbindungen gemäß den Ansprüchen 1 bis 6, bei denen
R für Phenyl steht, das durch 1 oder 2 Substituenten aus der Reihe -SO₃H, Halogen, -CN, C₁-C₄-Alkyl, -SO₂NH₂ substituiert sein kann.

8. Verbindungen gemäß den Ansprüchen 1 bis 7, bei denen A N oder 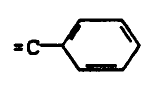 bezeichnet.

9. Verwendung der Verbindungen gemäß den Ansprüchen 1 bis 8 als Farbstoffe oder Pigmente.

10. Verwendung der Verbindungen gemäß den Ansprüchen 1 bis 8 mit Z ≠ -CN, W = N-H und X = Y = O als Pigmente.

## Claims

1. Heterocyclic compounds of the formula 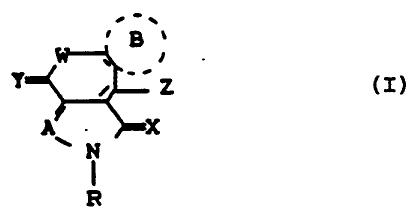 in which
R represents hydrogen, substituted or unsubstituted alkyl, substituted or unsubstituted cycloalkyl, substituted or unsubstituted aryl, substituted or unsubstituted hetaryl,
A represents C-R¹, N,
R¹ represents substituted or unsubstituted alkyl, substituted or unsubstituted cycloalkyl, substituted or unsubstituted aryl, substituted or unsubstituted hetaryl,
X, Y represent O, NH,
Z represents hydrogen, -CN, substituted or unsubstituted aryl, substituted or unsubstituted hetaryl,
W represents O, N-R⁵,
R⁵ represents hydrogen, substituted or unsubstituted alkyl, substituted or unsubstituted cycloalkyl, substituted or unsubstituted aryl, a substituted or unsubstituted heterocyclic radical and
B represents a substituted or unsubstituted carbocyclic-aromatic or heterocyclic ring onto which one or more further rings can be fused.

2. Compounds according to Claim 1 in which X = Y = O, W = N-R⁵, Z = H, -CN, substituted or unsubstituted aryl, A = N, C-alkyl, which can be substituted, C-aryl, which can be substituted, and B = a substituted aromatic ring, substituted pyrazole, pyrimidine or pyridine ring.

3. Compounds according to Claim 1 or 2 which in one of their tautomeric forms correspond to the formula 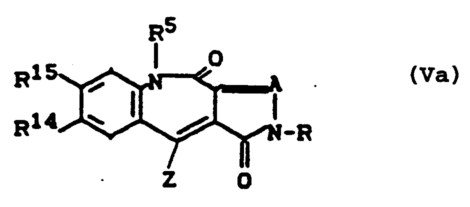 in which
R¹⁴, R¹⁵ represent hydrogen, halogen, OR³, NR⁵R⁶,
R³ represents hydrogen or has the meanings mentioned for R⁵ and
R⁶ has the meanings mentioned for R⁵.

4. Compounds according to Claim 1 or 2 which in one of their tautomeric forms correspond to the formula 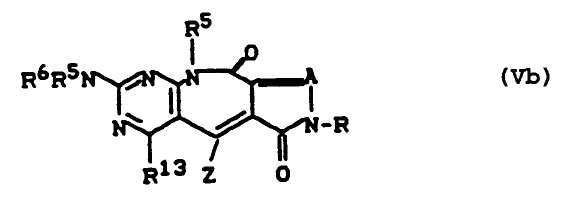 in which
R⁶ has the meanings mentioned for R⁵ and
R¹³ represents halogen or OR³ and
R³ represents hydrogen or has the meanings mentioned for R⁵.

5. Compounds according to Claim 1 or 2 which in one of their tautomeric forms correspond to the formula 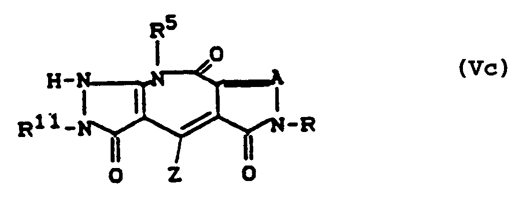 in which
R¹¹ is a substituted or unsubstituted aryl or a substituted or unsubstituted hetaryl radical.

6. Compounds according to Claim 1 or 2 which in one of their tautomeric forms correspond to the formula 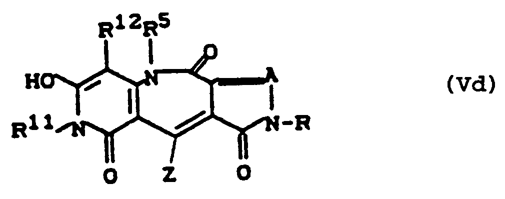 in which
R¹¹ is a substituted or unsubstituted aryl or a substituted or unsubstituted hetaryl radical,
R¹² is -CN, -COOR⁷, -CONR⁵R⁶,
R⁶, R⁷ have the meanings mentioned for R⁵.

7. Compounds according to Claims 1 to 6 in which
R represents phenyl which can be substituted by 1 or 2 substituents from the series consisting of -SO₃H, halogen, -CN, C₁-C₄-alkyl, -SO₂NH₂.

8. Compounds according to Claims 1 to 7 in which A represents N or

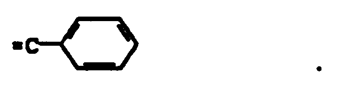

9. Use of the compounds according to Claims 1 to 8 as dyestuffs or pigments.

10. Use of the compounds according to Claims 1 to 8 in which Z ≠ -CN, W = N-H and X = Y = O as pigments.

## Revendications

1. Composés hétérocycliques de formule 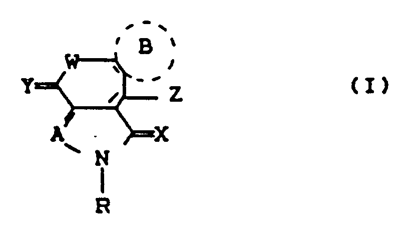 dans laquelle
R représente l'hydrogène, un groupe alkyle éventuellement substitué, un groupe cycloalkyle éventuellement substitué, un groupe aryle éventuellement substitué, un groupe hétaryle éventuellement substitué,
A représente C-R¹, N,
R¹ est un groupe alkyle éventuellement substitué, un groupe cycloalkyle éventuellement substitué, un groupe aryle éventuellement substitué, un groupe hétaryle éventuellement substitué,
X, Y représentent O, NH,
Z représente de l'hydrogène, un groupe -CN, un groupe aryle éventuellement substitué, un groupe hétaryle éventuellement substitué,
W représente O, N-R⁵,
R⁵ est de l'hydrogène, un groupe alkyle éventuellement substitué, un groupe cycloalkyle éventuellement substitué, un groupe aryle éventuellement substitué, un reste hétérocyclique éventuellement substitué et
B est un noyau carbocyclique-aromatique ou hétérocyclique éventuellement substitué, auquel un ou plusieurs autres noyaux peuvent être condensés.

2. Composés suivant la revendication 1, dans lesquels X et Y sont égaux et représentent O, W est un groupe N-R⁵, Z représente H, un groupe -CN, un groupe aryle éventuellement substitué, A représente N, un groupe C-alkyle qui peut être substitué, un groupe C-aryle qui peut être substitué et B est un noyau aromatique substitué, un noyau de pyrazole, de pyrimidine ou de pyridine substitué.

3. Composés suivant la revendication 1 ou 2, qui répondent sous l'une de leurs formes tautomères à la formule 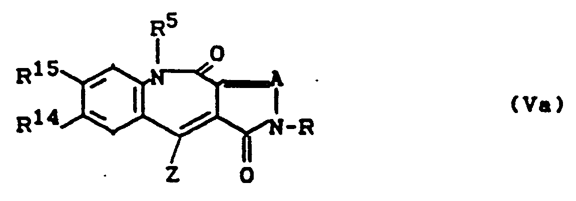 dans laquelle
R¹⁴, R¹⁵ représentent l'hydrogène, un halogène, un groupe OR³, NR⁵R⁶,
R³ est de l'hydrogène ou a les définitions mentionnées pour R⁵ et
R⁶ a les définitions mentionnées pour R⁵.

4. Composés suivant la revendication 1 ou 2, qui répondent sous l'une de leurs formes tautomères à la formule 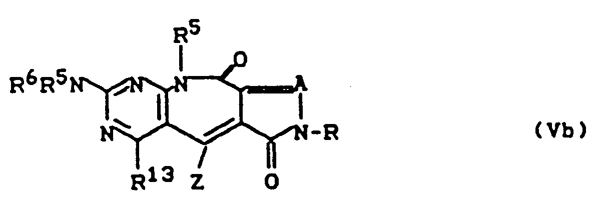 dans laquelle
R⁶ a les définitions mentionnées pour R⁵ et
R¹³ est un halogène ou un groupe OR³ et
R³ est de l'hydrogène ou a les définitions mentionnées pour R⁵.

5. Composés suivant la revendication 1 ou 2, qui répondent sous l'une de leurs formes tautomères à la formule 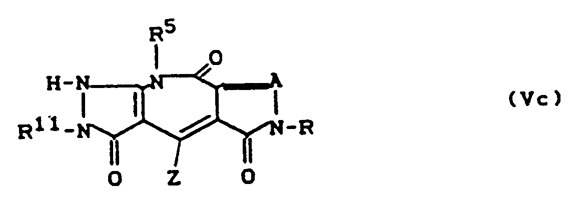 dans laquelle
R¹¹ est un reste aryle éventuellement substitué ou un reste hétaryle éventuellement substitué.

6. Composés suivant la revendication 1 ou 2, qui répondent sous l'une de leurs formes tautomères à la formule 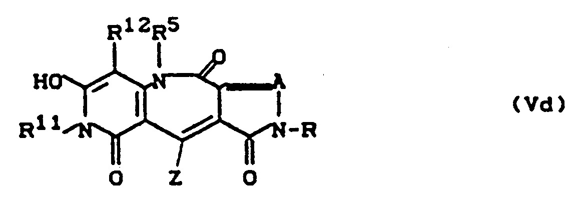 dans laquelle
R¹¹ est un reste aryle éventuellement substitué ou un reste hétaryle éventuellement substitué,
R¹² est un groupe -CN, -COOR⁷, -CONR⁵R⁶,
R⁶, R⁷ ont les définitions indiquées pour R⁵.

7. Composés suivant les revendications 1 à 6, dans lesquels
R est un groupe phényle qui peut être substitué par un ou deux substituants de la série -SO₃H, halogène, -CN, alkyle en C₁ à C₄, -SO₂NH₂.

8. Composés suivant les revendications 1 à 7, dans lesquels A représente N ou un groupe

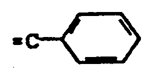

9. Utilisation des composés suivant les revendications 1 à 8 comme colorants ou pigments.

10. Utilisation des composés suivant les revendications 1 à 8, dans lesquels Z est différent de -CN, W est un groupe N-H et X et Y sont égaux et représentent O, comme pigments.
